# EUROPEAN PATENT APPLICATION

(11) **EP 1 891 941 A1**
(43) Date of publication of application: **27.02.2008**
(21) Application number: 06076563.3
(22) Date of filing: 11.08.2006
(51) Int. Cl.: A61K 9/16, A61K 9/00

(54) **Aqueous gels comprising microspheres**

(71) Applicant: OctoPlus Technologies B.V., 2333 CL Leiden (NL)
(72) Inventor: Van Tomme, Sophie Rolande, 3514 BH Utrecht (NL); Hennink, Wilhelmus Everhardus, 2743 CZ Waddinxveen (NL)
(74) Representative: van Loon, C.J.J.

(57) **Abstract**

The present invention provides viscoelastic gel compositions comprising microparticles which are capable of interacting with each other in a non-ionic manner, thus forming a gel network. The gel compositions may incorporate active agents and provide controlled release of such agents. The compositions are useful as injectable depot formulations of therapeutic compounds. In a further aspect, microparticles capable of gel formation in an aqueous environment are provided, as well as method of making viscoelastic gels based on the interaction of uncharged microparticles. Moreover, the invention discloses kits for making viscoelastic gel compositions and uses thereof.

## Description

### FIELD OF THE INVENTION

The present invention relates to pharmaceutical compositions in the form of viscoelastic gels. More in particular, it relates to aqueous gels which are particularly suitable for parenteral administration and/or which are capable of providing slow, sustained or controlled release of active compounds. The gels are also particularly suitable for tissue engineering. In further aspects, the invention relates to uses of such gels and methods of preparing them. Furthermore, kits are provided from which pharmaceutical gel compositions can be prepared.

More specifically, the present invention provides viscoelastic gel compositions comprising microparticles which are capable of interacting with each other in a non-ionic manner, thus forming a gel network. The gel compositions may incorporate active agents and provide controlled release of such agents. The compositions are useful as injectable depot formulations of therapeutic compounds. In a further aspect, microparticles capable of gel formation in an aqueous environment are provided, as well as method of making viscoelastic gels based on the interaction of uncharged microparticles.

### BACKGROUND OF THE INVENTION

Parenteral dosage forms with slow drug release properties have been developed to answer the need for improving the therapeutic use of drug substances which cannot be
- or are less suitable to be - administered orally due to their physicochemical properties, and which have a relatively short half life because of which they have to be injected frequently. Frequent injections are uncomfortable to patients, and if the injections have to be given by physicians or nurses, they are also rather costly. The experience of discomfort and pain may result in patient incompliance and may jeopardise the success of the therapy.

The number of drug substances which cannot be - or are less suitable to be - administered by the otherwise preferred oral route is presently increasing, primarily as a consequence of the recent advances of biotechnological research in the pharmaceutical area, which has lead to an increased number of highly potent peptide and protein drugs. Perhaps with the exception of some smaller peptides, however, these compounds are relatively unstable in gastrointestinal fluids and, more importantly, too large and hydrophilic as molecules to become absorbed through the intestinal mucosa to a substantial extent. For some of these drug substances, injectable or implantable controlled release formulations are being developed in order to lower the dosing frequency and thus reduce patient discomfort, and achieve a higher level of compliance and therapeutic success.

Parenteral controlled release dosage forms are usually in the form of macroscopic, solid single- or multiple-unit implants (such as polymeric rods and wafers), microparticle suspensions, and more recently also gels, including in situ forming gels. Drug-loaded solid implants are available as non-degradable polymeric, ceramic or metal devices which have to be surgically removed after the designated period of drug action, or as biodegradable polymeric forms which require no removal. An example for a non-degradable implant is Bayer's Viadur^{®}, which releases the peptide drug leuprolide over a period of one year. An example for a biodegradable implant is AstraZeneca's Zoladex^{®}, which is a polymeric rod capable of releasing the peptide drug goserelin over periods of one and three months, respectively.

A major disadvantage of implants is that they require very large needle diameters to be inserted, or in some cases even surgical incisions. Without premedication with a local anaesthetic, the implantation procedure would not be considered acceptable by a large number of patients. On the other hand, local anaesthesia can ensure that the procedure is painless.

Shortly after the market introduction of the first biodegradable implants, controlled release microparticles became available, such as Takeda's Lupron^{®} Depot formulations, which release leuprolide over periods of one, three, and four months, respectively. In order to inject such microparticles, they have to be suspended in an aqueous carrier. For stability reasons, however, depot microparticles cannot usually be stored as an aqueous suspension, but they have to be reconstituted from a dry powder.

Various designs of drug-loaded microparticles and methods for their preparation are described in E. Mathiowitz et al., Microencapsulation, in: Encyclopedia of Controlled Drug Delivery (ed. E. Mathiowitz), Vol. 2, (1999) 493-546, John Wiley & Sons, which is incorporated herein by reference.

Considering the required needle sizes for injecting microparticle suspensions (typically gauche 19 to 22), there is still a large difference to the relatively painless administration of a solution for injection. Smaller needle diameters are theoretically feasible for some microparticle preparations, but they bring about the risk of needle clogging in case the reconstitution is not carried out very carefully.

To overcome these problems, drug delivery scientists have in recent years begun to develop injectable gels which are capable of forming subcutaneous or intramuscular depots. In one of the concepts, gel formulations are designed which are highly shear thinning and thixotropic. By applying shear force prior to administration, the viscosity of these gels is substantially reduced, allowing for injection with a relatively small needle, whereas the gel strength is recovered slowly after administration. According to another concept, liquid compositions are formulated which, after administration, form gels in response to changes of their environment, such as pH, temperature, ionic strength. According to a third approach, liquid polymer formulations comprising a nonaqueous solvent are injected. Upon administration, the solvent diffuses away from the injection site, which leads to the precipitation of polymeric particles or to the formation of a gel.

Biodegradable injectable gels have been discussed in detail by A. Hatefi et al., Journal of Controlled Release 80 (2002), 9-28, which document is incorporated herein by reference.

Notwithstanding the recent progress in the design and manufacture of injectable gel formulations for controlled drug delivery, there remains a need for further improved gels, compositions, and preparation methods which overcome one or more of the disadvantages associated with presently available gels.

It is an object of the invention to provide improved aqueous gel compositions which are suitable, *inter alia,* for parenteral administration, convenient to use, and which have properties allowing their injection with acceptable needle sizes. It is another object of the invention to provide gel compositions comprising polymeric drug carriers which are safe and allow the tuning of release rates over periods of days, weeks, and months. In a further aspect, it is an object of the invention to provide kits and methods for making such gel compositions. Further objects will become apparent through the following description and examples.

### SUMMARY OF THE INVENTION

According to the invention, a viscoelastic aqueous gel composition is provided. The composition comprises microparticles comprising a crosslinked hydrophilic polymer. The microparticles are further characterised in that they have a core region and a surface region (or shell region) and in that at least the surface region of the microparticles comprises hydrophobic domains.

The invention is, *inter alia,* based on the discovery that certain types of polymeric microparticles, which are further described below, are capable of forming a viscoelastic gel when suspended in an aqueous medium. Surprisingly, gel formation can occur even in the absence of ionic charges on the surface of the microparticles. Furthermore, it has been found that the viscoelastic gels thus prepared are capable of entrapping bioactive compounds, either within the microparticles or in between the microparticles, and of releasing such compounds slowly over an extended period of time.

In a further aspect, the invention provides microparticles which are particularly useful for preparing such viscoelastic gels. The microparticles comprise a crosslinked hydrophilic polymer, and have a core region and a surface region, of which at least the surface region comprises hydrophobic domains. The hydrophobic domains are preferably formed by oligomeric side chains which are predominantly composed of monomeric units selected from L-lactide, D-lactide, glycolide, caprolactone, trimethylene carbonate, and combinations thereof. Furthermore, the invention provides a kit for the preparation of a viscoelastic aqueous gel composition, which kit comprises a solid composition comprising such microparticles.

The invention is also directed to the uses of the viscoelastic gel compositions of the invention, in particular in the manufacture of medicaments, diagnostic products, and tissue engineering products.

Moreover, the invention provides a method for making a viscoelastic aqueous gel composition. The method comprises the steps of (a) providing microparticles comprising a crosslinked hydrophilic polymer, and which have a core region and a surface region, of which at least the surface region comprises hydrophobic domains; (b) providing an aqueous liquid; and (c) combining the microparticles and the aqueous liquid to obtain the viscoelastic gel.

### DETAILED DESCRIPTION OF THE INVENTION

In a first principal aspect, the invention provides a viscoelastic aqueous gel composition. The gel composition comprises microparticles comprising a crosslinked hydrophilic polymer. The microparticles are further characterised in that they have a core region and a surface region, and that at least the surface region of the microparticles comprises hydrophobic domains.

As used herein, a viscoelastic gel is a semisolid material which is characterised by rheological properties which resemble, in part, the rheological behaviour of a viscous fluid and, also in part, that of an elastic solid. Macroscopically, a gel behaves like a solid upon the exertion of low shear force, and like a viscous fluid when the shear force exceeds a threshold which is defined as the yield point. According to a further definition, a gel is a system with a finite, usually rather small, yield stress.

It has been discovered by the inventors that certain types of microparticles, as described in more detail below, are capable of forming viscoelastic gels when suspended in an aqueous liquid. Surprisingly, gel formation can occur even in the absence of ionic charges on the surface of the microparticles, and it does not require very small particles sizes such as in the submicron range. Furthermore, the formation of the gel exhibiting viscoelasticity may occur in the absence of any other gel-forming agent which is dissolved or colloidally dispersed in the aqueous composition.

According to the invention, gels can be formed from microparticles comprising a crosslinked hydrophilic polymer, which microparticles have a core region and a surface region, and wherein at least the surface region of the microparticles comprises hydrophobic domains. In these gel compositions, the viscoelasticity is at least partially effected by the content of the microparticles.

Without wishing to be bound by theory, it is believed that hydrophobic interaction or other non-ionic interaction between the respective hydrophobic moieties at the surface of the microparticles can alone lead to gel formation if the microparticles also exhibit some degree of hydrophilicity.

An aqueous gel composition is also defined by the presence of water, usually of substantial amounts of water relative to the total mass of the composition. In a typical aqueous gel, the water forms a continuous phase in which the solid components which impart the gel strength to the system are dispersed. If the dispersion is colloidal, such as in a gelatin gel, such system could be considered monophasic, as no interphase between the colloid and the water can be determined. The gel compositions of the present invention, however, represent systems having at least two phases, i. e. a coherent aqueous phase and a dispersed, discontinuous, or incoherent solid or semisolid phase comprised of microparticles. Depending on other optional constituents, the compositions may comprise even more phases, such as a dispersed liquid phase or further dispersed solid phases.

The microparticles, as used herein, are substantially solid or semisolid particles having a weight- or volume average diameter in the region of about 0.1 to about 1.000 µm, but usually of about 0.1 to about 500 µm, and often from about 1 to about 500 µm, regardless of their composition, geometrical shape, or internal structure. For example, spherical microparticles, which are often referred to as microspheres or nanospheres, are included in the term microparticles, just as capsular structures, such as micro- or nanocapsules. Several other synonyms may exist to describe microparticles as defined above.

The microparticles, according the invention, comprise a crosslinked hydrophilic polymer. A polymer is defined by IUPAC nomenclature as a substance composed of macromolecules. Macromolecules or polymer molecules, in turn, are individual molecules of high relative molecular mass, the structure of which essentially comprises the multiple repetition of units derived, actually or conceptually, from molecules of low relative molecular mass, monomers. In common technical language, however, the term polymer is often used for both the substance and the macromolecules of which a polymeric substance is composed. It is this broader definition which is used herein, i.e. the term can refer to the substance or to a polymeric molecule or macromolecule, as applicable and evident from the respective context. Furthermore, a polymer may also refer to a copolymer, interpolymer, a mixture of (co)polymers, a crosslinked (co)polymer, or to polymeric networks.

Polymers are considered hydrophilic if, for example, they are water-soluble, i.e. in their non-crosslinked form. In this respect, water-soluble means soluble in water or in an optionally buffered aqueous medium substantially free of organic solvents a room temperature, exhibiting a solubility of at least about 1 mg/ml. This definition should be understood so a to comprise polymers which cannot be dissolved in water at room temperature, but at a higher temperature, as long as they remain in solution when cooled to room temperature. Alternatively, hydrophilic polymers may be not be soluble in water, but exhibit a significant degree of swelling in water, or in an optionally buffered aqueous medium at room temperature. In particular, a polymer which is not water-soluble is hydrophilic if it gains at least about 5% in volume or mass in such aqueous environment after equilibration. Another characteristic of many hydrophilic polymers is that they are better soluble in polar or even water-miscible organic solvents such as ethanol than in non-polar solvents such as hexane.

According to the invention, the microparticles comprised in the viscoelastic aqueous gel comprise at least one crosslinked hydrophilic polymer. As used herein, the crosslinks between the hydrophilic polymer molecules may be either of a physical or a chemical nature. Physical crosslinks may be formed e.g. through ionic interactions between oppositely charged groups of two or more polymer molecules, or between groups of two or more polymer molecules which are crosslinked by oppositely charged crosslinking agents. Other examples of physical crosslinks include (micro-)crystallites or (micro-)-crystalline domains, stereocomplexes, or any other type of non-covalent links between polymer molecules.

In contrast, chemical crosslinks are based on covalent bonds through which two or more polymer molecules are linked. Many types of chemical crosslinks and crosslinking agents are known in the art, a large number of which represent compounds having two or more reactive groups, such as described in Hennink et al., Advanced Drug Delivery Reviews 2002, 54:13-36, the disclosure of which is incorporated herein by reference.

The crosslinking, either intramolecularly or preferably intermolecularly of hydrophilic polymers typically leads to the formation of a so-called hydrogel. As used herein, such hydrogels are three-dimensional polymeric networks made by chemical or physical crosslinking of hydrophilic polymers. Physically crosslinked hydrogels are sometimes also termed physical hydrogels, whereas chemically crosslinked hydrogels are also called chemical hydrogels.

For the avoidance of confusion it should be emphasised that, within the context of the present invention, chemical or physical hydrogels are not necessarily identical with macroscopic, viscoelastic gels as defined above in terms of the latter's rheological properties. While it is possible that a hydrogel, which is defined by its three-dimensional polymeric network as described above, at the same time exhibits the typical rheological behaviour of a viscoelastic gel, this may not always be the case, and the respective definitions should be carefully distinguished. In particular, chemical hydrogels may not exhibit a yield point or become liquid under shear stress.

Among the hydrophilic polymers which are suitable for carrying out the present invention are, for example, hydrophilic polysaccharides, including native and derivatised polysaccharides, such as dextran, starch, amylose, amylopectin, cellulose, alginic acid, pectin, chitosan, hyaluronic acid, xanthan gum, pullulan, gellan gum, agar, carrageenan, dextrin, guar gum, carob gum, and inulin.

Among the particularly preferred polysaccharides are dextran derivatives, such as modified dextrans having polymerisable moieties like ethylenically unsaturated groups which can be reacted with each other by radical polymerisation. Very suitable are e.g. dextrans modified with hydroxyethyl methacrylic groups (dextran hydroxyethyl methacrylate, dexHEMA), in which the hydroxyethyl methacrylic groups are attached to the dextran backbone via carbonate linkers. Other suitable dextran derivatives include dextran hydroxypropyl methacrylate, dextran hydroxyethyl methacrylamide, and dextran hydroxypropyl methacrylamide.

It is generally known in the art how such dextran derivatives can be crosslinked in such a way that hydrogel microparticles are obtained. For example, such methods including useful reagents are described in WO 98/00170, WO 98/22093, WO 01/60339, and WO 03/035244, the disclosures of which are incorporated herein by reference.

Other suitable hydrophilic polymers are polypeptides, which can also be crosslinked physically or chemically. Examples of polypeptides which are useful to carry out the present invention include albumin, lysozyme, synthetic poly(amino acids), gelatin, collagen, poly(lysine) and related copolymers, poly(glutamic acid) and related copolymers, elastin, fibrin, casein, whey protein, lactoglobulin, lactalbumin, and soy protein. Again, the polypeptides may be used as substrates for crosslinking in their native or derivatised forms.

Further examples of hydrophilic polymers according to the invention include poly(acrylates), poly(acrylamides), poly(alkyl acrylates), poly(alkyl acrylamides), in particular poly(methacrylate), poly(hydroxyethyl methacrylate), poly(hydroxypropyl methacrylate), poly(hydroxyethyl methacrylamide), poly(hydroxypropyl methacrylamide); moreover poly(vinyl alcohol), poly(ethylene glycol), water soluble polyphosphazenes, and mixtures of any of the above.

As mentioned above, among the preferred hydrophilic polymers are dextrans derivatised with ethylenically unsaturated groups, such as dextran hydroxyethyl methacrylate, dextran hydroxypropyl methacrylate, dextran hydroxyethyl methacrylamide, and dextran hydroxypropyl methacrylamide. These dextran derivatives are preferably crosslinked by polymerising the acryl (or alkyl acrylic) groups to form a three-dimensional polymeric network, i.e. a chemical hydrogel, in such a way that microparticles are obtained.

According to a further preferred embodiment, the hydrophilic polymer comprises at least one bond which is hydrolysable under physiological conditions. In the context of the present invention, the terms "hydrolysable" and "hydrolysable under physiological conditions" are used interchangeably unless indicated otherwise, and relate to the capability of a molecule of becoming degraded by non-enzymatic hydrolysis in a native or simulated physiological fluid at body temperature. Typically, substantial hydrolysis occurs within hours, days, weeks, months, or a few years at the most, depending on the envisioned product application, e.g. the desired residence time of the gel or of the microparticles in a tissue, or the desired duration of release of an incorporated active agent.

The hydrolysable bonds may optionally be selected from carbonate, lactate, glycolate, succinate, and peptide bonds, representing the ester or amide bonds of the respective units or moieties. As used herein, a carbonate group or unit is a divalent CO₃ unit represented by the formula -O-(C=O)-O-. A lactate or glycolate group or unit is the divalent unit derived from lactic or glycolic acid as it is e.g. present in poly(lactic acid) or poly(glycolic acid), respectively. Similarly, a succinate group or unit is a divalent unit derived from succinic acid. Peptide bonds are the amide bonds between amino acids linked through their amino- and carboxylic acid groups, respectively. All these groups have been used in monomeric, oligomeric, or polymeric form in humans for a significant time, and therefore they can be considered safe.

In a preferred embodiment, the hydrolysable bonds are provided by a carbonate group between the main chain of the hydrophilic polymer and each polymerisable group. In another preferred embodiment, the side units comprise more than one hydrolysable group, such as a carbonate and one or two lactate units.

The introduction of a carbonate unit or one or more lactate, glycolate, or succinate units is described in further detail e.g. in WO 98/00170 and WO 98/22093, which are incorporated herein by reference. For example, in order to couple a polymerisable methacrylic group such as hydroxyethylmethacrylate (HEMA) - optionally linked to a monomeric or oligomeric lactate and/or glycolate unit - to a polysaccharide such as dextran, the terminal hydroxyl group of the substituent has to be activated. Preferably, the binding to the polysaccharide is effected by carbonyl-diimidazole (CDI) as coupling agent. However, also other activation methods can be used. For example, reaction of the hydroxyl function of the substituent with succinic anhydride, followed by activation of the formed carboxylic group using established methods (e.g. dicyclohexylcarbodiimide (DCC) activation). The latter method yields prepolymers in which only hydrolytically unstable ester bonds are present, which provide different degradation characteristics compared to the dextran derivatives synthesised with the CDI-method, in which both ester bonds and carbonate bonds are present.

The activated substituent, such as HEMA-oligo-lactate-carbonyl-imidazole, is subsequently coupled to the polysaccharide in a suitable aprotic solvent (such as DMSO). This can be done, optionally, in the presence of a catalyst, e.g. a base such as N,N-dimethylaminopyridine (DMAP) or triethylamine. However, it has been found by the inventors that it is also possible to conduct the coupling reaction without a catalyst, which has the advantage that no residual catalyst has to be specified or removed from the product before the prepolymer can be used as a starting material for medicinal products. The degree of substitution (i.e. number of moles of methacrylate groups containing prepolymer per 100 moles glucose units of dextran) can be tailored by the ratio of HEMA-containing prepolymer versus polysaccharide in the reaction mixture.

A further feature of the viscoelastic gel of the invention and of the microparticles comprised therein is that the microparticles have a core region and a surface region, and that at least the surface region comprises hydrophobic domains.

As used herein, the terms "surface region"and "core region" refer to the respective local positions in the microparticles, e.g. either in the superficial region near the outer surface (say, the outer 10% volume percent) of the microparticles or beneath this region. In contrast, the terms should not be interpreted as implying that the composition or structure of the core and surface regions are necessarily different. It is believed that the composition of the surface region of the microparticles plays an important part in the gel-forming capabilities of the microparticles; this does however not exclude that the microparticles are substantially homogeneous.

As used herein, a domain is understood as a region of or within a microparticle or within the surface region of a microparticle having a certain physical property, in the present case a degreeof hydrophobicity, which differs from that of other regions of the same microparticle or surface region.

The hydrophobic domains may represent covalently attached domains, substituents, blocks or side chains which are more hydrophobic, or less hydrophilic, than e.g. the main chains of the crosslinked hydrophilic polymer. In this context, the expressions "more hydrophobic" and "less hydrophobic" mean that a compound predominantly consisting of the chemical structure(s) which form(s) the domain would have a substantially lower water-solubility or swellability, as defined herein, than the hydrophilic polymer in non-crosslinked form. In particular, a substantially lower solubility or swellability refers to a solubility or swellability which is not more than about half of the reference solubility or swellability. Furthermore, a compound predominantly consisting of the chemical structure(s) which form(s) the hydrophobic domains is typically better soluble in an organic solvent such as dichloromethane or ethyl acetate than in water.

Alternatively, the hydrophobic domains may be represented by the hydrophobic regions of amphiphilic ions which are ionically bound to the microparticles. Examples of potentially suitable amphiphilic ions include cationic, anionic, and zwitterionic surfactants such as sodium dodecyl sulfate, ammonium lauryl sulfate, other alkyl sulfate salts, sodium laureth sulfate, alkyl benzene sulfonate, salts of fatty acids, cetyl trimethylammonium bromide, hexadecyl trimethyl ammonium bromide, other alkyltrimethylammonium salts, cetyl pyridinium chloride, polyethoxylated tallow amine, benzalkonium chloride, dodecyl betaine, dodecyl dimethylamine oxide, cocamidopropyl betaine, coco amphoglycinate, and phospholipids such as lecithin, purified phosphatidylcholins or fractions thereof.

According to a further alternative embodiment, the hydrophobic domains are formed by the hydrophobic regions of non-ionic amphiphilic molecules which are adsorbed to the microparticles, forming their outer surface or a part of it. Examples of potentially suitable non-ionic amphiphilic molecules include alkyl poly(ethylene oxide), alkyl polyglucosides such as octyl glucoside or decyl maltoside, fatty alcohols such as cetyl alcohol or oleyl alcohol; cocamide MEA, cocamide DEA, cocamide TEA; and amphiphilic block polymers such as poloxamer.

In one of the particularly preferred embodiments, the hydrophobic domains are covalently attached to the microparticles, i.e. to the crosslinked hydrophilic polymer which is a major constituent of the microparticles. According to this embodiment, the domains are hydrophobic substituents, such as alkyl-, acyl, or aryl groups.

Without wishing to be bound by theory, it is believed that in the composition of the present invention, the formation of the viscoelastic gel occurs through the hydrophobic domains of neighbouring microparticles which interact with each other non-covalently and non-ionically in an aqueous environment, either by hydrophobic interaction, hydrogen bonding, stereocomplex formation, or by any other type of non-covalent and non-ionic interaction, depending on the specific hydrophobic domains which are present in the surface region of the microparticles.

In particular, it has been found that hydrogel microparticles whose surface region comprises oligomeric or polymeric grafts or side chains composed of units such as lactide or glycolide appear to be capable of interacting with the respective domains of neighbouring microparticles of the same type in such a way that gel formation occurs in an aqueous environment. This is particularly surprising in view of the fact that neither the hydrogel microparticles alone, i.e. without the oligolactide or -glycolide side chains, nor microparticles composed of polymers constructed entirely from the hydrophobic lactide and/or glycolide units exhibit any noteworthy capability of gel formation in water.

More in detail, very suitable hydrophobic domains are created in the surface region of hydrogel microparticles by grafting such microparticles with oligomeric or polymeric side chains which are predominantly composed of monomeric units selected from L-lactide, D-lactide, glycolide, and mixtures thereof. As used herein, a side chain is oligomeric if it comprises at least two monomeric units. In one of the preferred embodiments, the side chains each comprise from 2 to about 20 lactide or glycolide units, in particular from about 3 to about 16, or from about 4 to about 14 units, respectively.

The degree of polymerisation may be relatively constant within a species of microparticles, or it may vary around an average degree of polymerisation. In one of the presently preferred embodiments, the side chains are substantially monodisperse. In another embodiment, they exhibit the degree of polydispersity which typically results from synthesising oligolactides and/or oligoglycolides without subsequent fractionisation.

The side chains of a microparticle, or the hydrophobic domains in more general terms, may be of one type, or they may represent a mixture of two or more chemically different structures. For example, a microparticle may simultaneously comprise oligolactide and oligoglycolide grafts, or it may comprise oligo-D-lactide and oligo-L-lactide side chains.

As mentioned above, it is necessary that the hydrophobic domains are present in the surface region of the microparticles, but they may or may not be present in the core region. Depending on the actual hydrophobicity of the domains, e.g. on the type of monomeric unit and the degree of polymerisation, it may be advantageous to design the microparticle core region such as to comprise relatively less hydrophobic domains than the surface region, or even to select a method of preparation which will effect that only the surface region, but not the core region, comprises hydrophobic domains. For example, if the hydrophilic polymer is first crosslinked to obtain the microparticles and subsequently the microparticles are substituted to have the hydrophobic domains, the resulting microparticles will typically exhibit a more densely substituted surface, whereas a method involving the crosslinking of the hydrophilic polymer after it is coupled with the hydrophobic substituent(s) will more typically lead to particles with a similar density of hydrophobic domains in the core and surface regions, respectively.

The degree of substitution may be chosen within wide ranges in consideration of the desired gel strength of the viscoelastic gel composition, the selected hydrophilic polymer, the selected hydrophobic domains, the relative size of the hydrophobic domains (e.g. the degree of polymerisation in the case of oligomeric side chains), and the size of the microparticles.

For example, in the case of the selection of a crosslinked polysaccharide such as a dextran derivative for the hydrophilic polymer and the selection of oligolactide, oligoglycolide and/or oligolactide-co-glycolide as hydrophobic domains, a preferred degree of substitution is in the range from about 0.1 to about 30. In this context, the degree of substitution is the number of side chains or grafts per 100 monosaccharide units of the polysaccharide, i.e. per 100 glucopyranose units in the case of dextran. In another preferred embodiment, the degree of substitution is in the range from about 0.5 to about 20, or from about 1 to about 15, respectively. For the purpose of calculating the number of monosaccharide units, all monosaccharide units are included, without distinction between the core region and the surface region.

In addition to the hydrophobic domains, the microparticles may also comprise other domains comprising ionic charges, such as to be capable of interacting ionically with oppositely charged domains of neighbouring microparticles. Presently preferred are microparticles without ionic charges.

According to another particularly useful embodiment of the invention, the microparticles and their hydrophilic domains are selected to be capable of interacting with each other in such a way that stereocomplexes are formed. A stereocomplex may be formed between a hydrophobic domain of a first microparticle and the hydrophobic domain of a second microparticle if the two respective domains have a substantially opposite chirality or stereochemical configuration. For example, a stereocomplex may occur between an oligo-L-lactide side chain of a first microparticle and an oligo-D-lactide side chain of a second particle. Of course, stereocomplexes are also possible between two side chains of the same microparticle if the respective stereoconfigurations are present; however, such intraparticular stereocomplexes are not believed to contribute significantly to the formation of a viscoelastic gel composition as defined herein.

The stereochemical configuration is the arrangement of atoms in a molecule (or in a molecular domain) in three-dimensional space, especially with regard to the differences between enantiomers. If an organic molecule includes a carbon atom with four different substituents, these substituents can be arranged in two different ways, resulting in two different molecules called enantiomers, which usually have the same chemical properties, but which may differ in some physical properties. Typically, enantiomers have optical activity; that is, they rotate polarised light. The member of the pair of enantiomers which rotates polarised light clockwise is specified in formulas with a D for dextrorotatory, and the enantiomer which rotates polarised light counterclockwise is levorotatory or L. The phenomenon is also referred to as chirality.

A prerequisite of stereocomplex formation according to the invention is the presence of chiral carbon atoms. Moreover, it is required that enantiomerically enriched molecular regions or, in the present case, of enantiomerically enriched hydrophobic domains, grafts, or side chains, are present. It is not required according to the invention that such enantiomerically enriched domains are exclusively constructed from monomeric units which have the same configuration, i.e. either the L- or D-configuration. However, one configuration should be dominant in a domain or side chain which is designed to undergo stereocomplexation. In this context, a configuration is dominant if it is at least about twice as frequent in a hydrophobic domain as the opposite stereochemical configuration. More preferably, substantially all monomeric units of an enantiomerically enriched side chain have the same chirality.

To avoid misunderstandings, it is not required that all hydrophobic domains of all microparticles in a gel composition must be chiral or enantiomerically enriched to enable stereocomplex formation between the microparticles. For example, the presence of non-chiral or non-enriched side chains next to the enantiomerically enriched side chains will generally not prevent the formation of stereocomplexes.

According to a further preferred embodiment, the gel composition of the invention comprises at least a first and a second species of microparticles, both species having a core region and a surface region, and both species comprising a crosslinked hydrophilic polymer. At least the surface region of the microparticles of both species comprise oligomeric or polymeric side chains which are predominantly composed of monomeric units selected from L-lactide, D-lactide, combinations thereof, and combinations of any of these with caprolactone, trimethylene carbonate, and glycolide, which units are selected in such a way that oligomeric side chains of the first species of microparticles are capable of forming stereocomplexes with oligomeric side chains of the second species of microparticles.

For example, the gel composition may comprise two species of microparticles, both of which comprise a crosslinked polysaccharide such as crosslinked dextran hydroxyethylmethacrylate. Furthermore, both species of microparticles may be - at least in their surface region - substituted with oligomeric chains of e.g. lactide units. In this example, the microparticles of the two species would differ from each other in that at least some of the oligolactide side chains of the first species are enantiomerically enriched such that either the D- or the L-configuration is dominant, whereas in at least some of the oligolactide side chains of the second species the opposite configuration is dominant. Preferably, within each species of microparticles, there is only one dominant stereochemical configuration, and side chains which are enantiomerically enriched to have the opposite chirality are substantially absent.

According to another example, the gel composition of the invention comprises two species of microparticles based on crosslinked dextran hydroxyethylmethacrylate whose surface region is substituted with oligolactide side chains, wherein substantially all lactide units of the first species of microparticles have a first (D- or L-) configuration, and wherein substantially all lactide units of the second species of microparticles have the configuration which is of opposite chirality to the first configuration.

In another example, the gel comprises two species of microparticles based on a crosslinked dextran derivative other than dextran hydroxyethylmethacrylate, wherein the surface regions are substituted with oligolactide side chains. Again, substantially all lactide units of the first species of microparticles have a first (D- or L-) configuration, and substantially all lactide units of the second species of microparticles have the configuration which is of opposite chirality to the first configuration.

Obviously, there are many further options of combining microparticles based on a crosslinked hydrophilic polymer having superficially attached chiral substituents or side chains, in such a way that stereocomplex formation can occur between neighbouring particles, thus leading to non-covalent interaction and the formation of a viscoelastic gel.

Optionally, a gel composition may comprise a third species of microparticles, for example microparticles having a similar composition as the first two species except that their side chains are not enantiomerically enriched. In this case, it is preferred that the major effect on the formation of the viscoelastic gel is exerted by the first two species, i.e. the microparticles with hydrophobic domains having opposite chirality.

The microparticles which are capable of gel formation as described above should have an average diameter from about 0.1 to about 500 µm. More preferably, the average diameter as determined by laser diffraction is in the range from about 1 to about 150 µm, in particular from about 1 to about 50 µm, or from about 5 to about 40 µm, respectively.

If the microparticles or the gel composition is designed to deliver one or more antigens for the purpose of vaccination, relatively small microparticle diameters may also be highly useful. In these embodiments, the average diameter is preferably selected in the range from about 0.1 µm to about 500 µm, or from about 0.5 µm to about 150 µm, respectively.

The content of microparticles should be selected to achieve the desired gel strength or storage modulus of the aqueous gel composition in consideration of the type of particles which have been selected. Typically, the content of microparticles in the gel composition is in the range from about 5 to more than about 50 wt.-%, wherein the weight of the microparticles is their weight in the dried state. More preferably, the microparticle content is in the range from about 5 to about 30 wt.-%.

In some further preferred embodiments in which the microparticles are based on a crosslinked dextran derivative and the hydrophobic domains are oligolactide chains having a preferred degree of polymerisation and a preferred degree of substitution as described above, the microparticle content is from about 10 wt.-% to about 20 wt.-%.

The gel composition is particularly useful for the delivery of an active compound to a human or animal in need thereof. Thus, the composition preferably comprises one or more bioactive compounds. As used herein, a bioactive compound is any chemical or biological substance or mixture of substances which is useful for the diagnosis, prevention or treatment of diseases, symptoms, and other conditions of the body, or for influencing a body function. In this context, the terms "active" and "bioactive" may be used interchangeably. Other terms which may be used are active agent, active ingredient, drug substance, and the like.

Among the preferred active compounds are those which are used in chronical or long-term treatment regimen and/or which have a low oral bioavailability, such as hormones, growth factors, hormone antagonists, antipsychotics, antidepressants, cardiovascular drugs, and the like. In another aspect, a preferred class of active compounds is that of peptides and proteins, in particular proteins, which can be delivered effectively with the gel compositions of the invention, providing drug release over extended time periods, thus eliminating the need for the frequent injection of these compounds.

Among the preferred peptides and proteins are erythropoetins, such as epoetin alpha, epoetin beta, darbepoetin, haemoglobin raffimer, and analogues or derivatives thereof; interferons, such as interferon alpha, interferon alpha-2b, PEG-interferon alpha-2b, interferon alpha-2a, interferon beta, interferon beta-1a and interferon gamma; insulins; antibodies, such as rituximab, infliximab, trastuzumab, adalimumab, omalizumab, tositumomab, efalizumab, and cetuximab; antibody fragments; blood factors such as alteplase, tenecteplase, factor VII(a), factor VIII; colony stimulating factors such as filgrastim, pegfilgrastim; growth hormones such as human growth factor or somatropin; interleukins such as interleukin-2 and interleukin-12; growth factors such as beclapermin, trafermin, ancetism, keratinocyte growth factor; LHRH analogues such as leuprolide, goserelin, triptorelin, buserelin, nafarelin; antigens, vaccines; etanercept, imiglucerase, drotrecogin alpha.

Antigens and vaccines, whether these constitute peptides, proteins, lipoproteins, polysaccharides, live or inactivated microorganisms or viruses, or subunits thereof, including any adjuvants capable of increasing an immune response triggered by an antigen or vaccine, form another preferred group of active compounds for whose delivery the present invention is useful.

Other preferred active compounds are polysaccharides and oligo- or polynucleotides, DNA, RNA, iRNA, hormones, cytostatic agents, cytotoxic agents, antibiotics, and living cells. Another class of preferred active compounds comprises drug substances acting on the central nervous system, even if they are small molecules and orally bioavailable, for example risperidone, zuclopenthixol, fluphenazine, perphenazine, flupentixol, haloperidol, fluspirilen, quetiapine, clozapine, amisulprid, sulpirid, ziprasidon, etc.

Alternatively, the active compound may be a native living cell, a fragment of a living cell, a modified cell, or a plurality of cells. Encapsulated or immobilized cells can potentially be injected or implanted to replace physiological functions which are absent in a patient due to a specific disease or condition. For example, diabetes patients could be treated with gel-encapsulated Langerhans cells which can produce and secrete insulin. In this application, both the living cells and the insulin could be considered as the active compound.

In a further embodiment, the active compound may be incorporated in the form of drug-loaded colloidal carriers, such as nanoparticles, nanocapsules, liposomes, lipoplexes, lipid complexes, iscoms, polyplexes, solid lipid nanoparticles, virosomes, or drug conjugates.

Two principal options exist with respect to the incorporation of the active compound into the aqueous gel composition. In a first preferred embodiment, the active compound is at least in part incorporated in the microparticles; and preferably, at least a fraction of the microparticles present in the composition is loaded with active compound. If two or more active compounds are incorporated in the composition, at least one of them, and optionally all of them, are accommodated within microparticles.

Based on this embodiment, release rates can be tailored through adjusting the hydrolysability of the polymers forming the microparticles. For example, if the active compound is a macromolecular substance, such as a protein, and the microparticles are hydrogels representing water-swollen, porous, three-dimensional polymeric networks, drug release is likely to take place primarily by degradation and erosion of the hydrogel, as the pores of the non-degraded hydrogel will typically be too small to allow for drug release by diffusion.

The content of the active compound relative to the microparticles may typically be selected in the range from about 0.1 to about 25 wt.-%. In another embodiment, the content is in the range from about 0.5 to about 10 wt.-%. In the case that two or more active compounds are incorporated, these ranges should be applied to the content of the combined compounds.

According to a second preferred embodiment, some or all of the active compound is incorporated into the aqueous gel phase, but not within the microparticles. Depending on the specific composition of the gel and on the physical properties of the active compound, it is quite possible to achieve controlled release of the compound even without its incorporation within the particles, such as over a period of one or more days, weeks, or even months. As used herein, controlled release should not be understood to refer to a specific type of release profile, but more generally to slow release, prolonged release, sustained release, or extended release.

The extraparticular incorporation of the active compound may have advantages with respect to stability. Many of the preferred active compounds as listed above are rather sensitive to heat, organic solvents, and various chemical reagents. On the other hand, these potentially harmful factors and agents are often required in the manufacture of microparticles. By preparing microparticles which are free of active compound and mixing them with water, the active compound, and further optional ingredients, to obtain the aqueous gel composition of the present invention, the degradation of the compound during manufacture may be largely avoided.

In the case of extraparticular incorporation, the content of the active compound relative to the aqueous gel composition is typically selected in the range from about 0.01 to about 20 wt.-%. In a further embodiment, the content is in the range from about 0.1 to about 10 wt.-%. In the case that two or more active compounds are incorporated, these ranges should be applied to the content of the combined compounds.

Regardless of how the active compound is incorporated, i.e. whether inside the microparticles or not, it is preferred that it is released over a period of at least one day, such as in the range from about one day to about six months, or in the range from about 1 week to about 3 months, or in the range from about 10 days to about 1 month. As used herein, the duration of release is understood as the time required for the release of at least about 80 %, preferably of at least about 90 %, of the incorporated active compound into a physiological fluid at about 37 °C.

The gel composition of the invention exhibits viscoelastic properties, which means that it is a semisolid material which is characterised by rheological properties which resemble, in part, the rheological behaviour of a viscous fluid and, also in part, that of an elastic solid, as described above. One of the key parameters which describes a gel is the yield point, or yield stress. This may be defined as the stress at which a gel begins to plastically deform. Prior to the yield point the gel will deform elastically and will return to its original shape when the applied stress is removed. Other parameters to describe a viscoelastic gel include the storage modulus and the loss modulus. Suitable methods to determine these parameters are described e.g. in the working examples (see e.g. Examples 3 and 4) below. Further guidance on the principles and modes of determining these parameters and other viscoelastic properties is found in A. Franck, Viscoelasticity and dynamic mechanical testing, TA Instruments, Application Note AN004.

The yield stress or yield point of the gel composition is typically higher than about 5 Pa, and preferably substantially higher, such as e.g. at least about 50 Pa, or at least about 100 Pa. The values should be understood as measured at room temperature. Depending on the particular product application, the composition should be adjusted to even higher yield stress values, such as about 200 Pa or more, or about 500 Pa or more. In further preferred embodiments in which the gel comprises at least two species of microparticles whose hydrophobic domains are capable of forming stereocomplexes with each other, the yield stress is at least about 50 Pa, or in the range from about 50 to about 2,000 Pa, or in the range from about 100 to 1,000 Pa, respectively, in particular if the composition is intended for use as an injectable depot formulation providing controlled release of an active agent. If the intended use relates to tissue engineering, even higher yield points may be selected, such as at least about 200 Pa, or in the range from about 200 to 5,000 Pa, respectively.

Another related parameter which describes the quantity of the elastic component of the viscoelastic behaviour of a gel is the storage modulus, usually abbreviated as G'. Again, the higher the storage modulus, the stronger and the more "solid-like" a gel would appear. According to the present invention, it is preferred that the gel composition exhibits a storage modulus of at least about 50 Pa, and in particular of at least about 100 Pa. In further preferred embodiments, the storage modulus is adjusted to a value of at least about 500 Pa or at least about 1,000 Pa, or at least about 2,000 Pa, respectively. Again, the values should be understood as measured at room temperature. For certain product applications such as tissue engineering, even higher G' values may be selected, and are in fact achievable based on the teachings of the invention, such as in the range from about 2,000 to about 30,000 Pa.

To adjust the storage modulus to the desired value with an eye on the intended product application - for example in consideration of a desired local tissue residence time after subcutaneous injection - is easily possible by following the guidance provided herein, in particular by appropriately selecting the chemical nature of the hydrophobic domains, their stereochemical configuration, their size or chain length, and the microparticle content in the gel.

For example, if a higher storage modulus is desired for a gel composition comprising microparticles from a crosslinked polysaccharide such as dextran hydroxyethylmethacrylate comprising hydrophobic domains in the form of oligolactide or oligoglycolide side chains, the following means to achieve this should be considered: (a) an increase of the microparticle content, either into or within the preferred ranges given herein; (b) an increase of the relative quantity of hydrophobic domains, i.e. of the degree of substitution, either into or within the preferred ranges; (c) an increase of the size of hydrophobic domains, i.e. the degree of polymerisation of the lactide or glycolide units, either into or within the preferred ranges; (d) the selection of two species of microparticles having enantiomerically enriched oligolactide side chains with opposite chirality, so that stereocomplex formation can occur between microparticles rather than hydrophobic interaction and/or hydrogen bonding only.

Of course, it is also possible to increase the gel strength by incorporating a gel-forming agent as an additional excipient into the gel composition in dissolved or colloidally dispersed form. Examples of gel-forming agents include water-soluble or water-dispersible native and derivatised polysaccharides such as dextran, starch, amylose, amylopectin, cellulose, methyl cellulose, hydroxypropyl cellulose, hypromellose, alginic acid, pectin, chitosan, hyaluronic acid, xanthan gum, pullulan, gellan gum, agar, carrageenan, dextrin, guar gum, carob gum, and inulin. Other gel-forming agents include certain proteins such as albumin, lysozyme, synthetic poly(amino acids), gelatin, collagen, poly(lysine) and related copolymers, poly(glutamic acid) and related copolymers, elastin, fibrin, casein, whey protein, lactoglobulin, lactalbumin, and soy protein. Alternatively, synthetic gel-forming polymers may be used, such as poly(acrylates), poly(acrylamides), poly(alkyl acrylates), poly(alkyl acrylamides), in particular poly(methacrylate), poly(hydroxyethyl methacrylate), poly(hydroxypropyl methacrylate), poly(hydroxyethyl methacrylamide), poly(hydroxypropyl methacrylamide); moreover poly(vinyl alcohol), poly(ethylene glycol), water soluble polyphosphazenes etc.

However, it is preferred that the viscoelastic behaviour of the composition is, at least predominantly, caused by the microparticles comprised in the composition. In another embodiment, a dissolved or colloidally dispersed gel-forming agent other than the microparticles is absent.

The gel composition can be adapted for topical, oral, rectal, vaginal, ophthalmic, or pulmonary administration; preferably, they are adapted for parenteral or topical administration. As used herein, parenteral administration includes any invasive route of administration, such as subdermal, intradermal, subcutaneous, intramuscular, locoregional, intratumoral, intraperitoneal, interstitial, intralesional, intra-articular, with some less preference in the context of this invention also intravenous, intraarterial etc. The presently most preferred parenteral routes of administration of the gel compositions are subcutaneous, intramuscular, and intratumoral.

Topical administration refers to administration to the skin or to a mucosal surface. Pulmonary administration includes oral or nasal inhalation by means of, e.g. a nebuliser, a powder inhaler, or a metered dose inhaler.

When the invention is used for tissue engineering applications, the sites of injection or implantation may of course differ widely, depending on the specific tissue or organ whose function is to be replaced or augmented. As mentioned above, in these applications it is the living cells that take the place of the active compound.

Being adapted for parenteral administration also means that such gel compositions are formulated and processed to meet the requirements of parenteral dosage forms. Such requirements are, for example, outlined in the major pharmacopoeias. In one aspect, the composition, or its premixes or the kits from which the composition is made prior to administration, must be sterile. As used herein, the term "sterility" is to be defined according to the usual pharmaceutical meaning. It is understood as the absence of germs which are capable of reproduction. Sterility is determined with suitable tests which are defined in the relevant pharmacopoeias.

In another aspect, the excipients must be selected to be safe and tolerable for parenteral administration. In a further aspect, the compositions are formulated to be relatively isotonic (or isoosmotic), such as in the region of about 150 to 500 mOsmol/kg, and preferably in the region of about 250 to 400 mOsmol/kg. Furthermore, the pH should be approximately in the physiological range in order to avoid pain and local intolerance upon injection. Preferably, the pH of the composition is in the region of about 4 to 8.5, and more preferably in the region of about 5.0 to 7.5.

Further excipients may be incorporated in the gel composition as needed, such as stabilisers, bulking agents, matrix forming agents, lyophilisation aids, antioxidants, chelating agents, preservatives, solvents, cosolvents, surfactants, osmotic agents, acidic or alkaline excipients for adjusting the pH, etc.

Preferably, the gel composition of the invention is adapted to be capable of being administered with a needle of 17 gauge or higher, and more preferably with a needle of 20 gauge or higher, and even more preferably with a needle of 22 gauge, 24 gauge, 26 gauge, or higher.

As used herein, the capability of being administered refers to rheological properties which allow the injection with the specified needle type without requiring an injection force of more than about 25 N. More preferably, the rheological properties are adapted, and a needle size selected, to enable injection with a force of no more than about 20 N, and even more preferably with an injection force of no more than about 15 N, to allow the administration also to be performed by physicians, nurses, or patients who are not particularly sinewy.

The adaptation of the gel composition for parenteral administration is particularly important if the composition is to be used as a medicament, or drug product. In general, the gel composition may be used for various pharmaceutical and non-pharmaceutical applications including, without limitation, cosmetics, food products or food additives, research, diagnostics, tissue engineering, medical devices, etc. In one of the preferred embodiments, the gel composition comprises a therapeutic active compound and is used as a medicinal or drug product.

The use as a drug product may involve topical, oral, rectal, vaginal, ophthalmic, or pulmonary administration. According to a preferred embodiment, the gel composition is adapted and used for parenteral or topical administration. As used herein, parenteral administration includes any invasive route of administration, such as subdermal, intradermal, subcutaneous, intramuscular, locoregional, intratumoral, intraperitoneal, interstitial, intralesional, intra-articular, with some less preference in the context of this invention also intravenous, intraarterial etc. The most preferred routes of administration of the gel compositions are subcutaneous, intramuscular, and intratumoral.

When the invention is used for tissue engineering applications, the sites of injection or implantation may of course differ widely, depending on the specific tissue or organ whose function is to be replaced or augmented. As mentioned above, in these applications it is the living cells that may represent the active compound.

Again, one of the preferred uses is that of a medicament which is administered parenterally. Preferably, the medicament is a depot formulation which releases its incorporated active ingredient over a period of at least one day. In further embodiments, the period of release is from about 2 days to about 6 months, or from about half a week to about 4 months, or from about 1 week to about 3 months, such as about 2 weeks, about 4 weeks, about 1 month, about 2 months, about 3 months, or about 4 months.

In a further aspect, the invention is directed to the microparticles themselves which have been described above as essential components of the viscoelastic gel compositions. In its broadest scope, the invention provides a plurality of microparticles which are substantially free of ionic charges, but adapted to be capable of forming a viscoelastic gel in an aqueous environment. In particular, the microparticles comprise a surface region with hydrophobic domains, and are capable of forming a viscoelastic gel by virtue of hydrophobic interaction between hydrophobic domains, optionally also by hydrogen bonding. In a further embodiment, the microparticles comprise a surface region with hydrophobic domains, and are capable of forming a viscoelastic gel through the formation of stereocomplexes between the hydrophobic domains of neighbouring particles.

In one of the preferred embodiments, the microparticles of the invention have a core region and a surface region, and they comprise a crosslinked hydrophilic polymer; and at least the surface region of the microparticle comprises hydrophobic domains formed by oligomeric side chains which are predominantly composed of monomeric units selected from L-lactide, D-lactide, glycolide, and combinations thereof. With respect to the hydrophilic polymer, the hydrophobic domains, optionally incorporated active agents and other features of the microparticles, the same guidance and preferences should be observed as have been given above in the context of the disclosure of the aqueous gel composition.

In yet a further aspect, the invention provides a method for making a viscoelastic aqueous gel composition. The method comprises the steps of:
(a) providing microparticles having a core region and a surface region, wherein the microparticles comprise a crosslinked hydrophilic polymer, and wherein at least the surface region of the microparticles comprises hydrophobic domains;
(b) providing an aqueous liquid; and
(c) combining said microparticles and said aqueous liquid to obtain the viscoelastic gel.

In this method, the microparticles should be designed or selected with respect to their features as outlined above. It should be noted that the step of providing the microparticles may involve that the microparticles are not provided in an isolated form, but in the form of a liquid or solid composition which may further comprise one or more carriers or suitable excipients. For example, the microparticles may be provided as a lyophilised solid component further comprising one or more bulking agents, lyophilisation aids, stabilisers, surfactant, suspension agents and the like. Alternatively, the microparticles may be provided as a free flowing powder component, optionally comprising one or more excipients as appropriate.

The aqueous liquid to be provided should be selected according to the desired gel properties and the intended use of the resulting gel composition. For example, if the gel composition is designed for injectable use, the aqueous liquid carrier should be sterile and composed of excipients that are well tolerated upon injection. As used herein, an aqueous liquid is a liquid whose predominant liquid constituent is water. The presence of one or more other solvents is optional; in this case, the other solvent(s) should be selected from water-miscible organic solvents that are suitable for injection, such as ethanol, glycerol, propylene glycol, polyethylene glycol, N-methyl pyrrolidone (NMP), dimethylformamide, dimethyl sulfoxide, or tetrahydrofuran. The incorporation of such solvents or co-solvents should be contemplated when an essential component of the gel composition must be dissolved or solubilised in the aqueous liquid, but exhibits poor aqueous solubility. In one of the preferred embodiments, the aqueous liquid comprises water as the only liquid constituent.

The liquid may comprise one or more suitable excipients in dissolved form, as appropriate. Depending on the intended use, such excipients may be selected according to common practise. Examples of potentially suitable excipients include stabilisers, antioxidants, preservatives, cosolvents, surfactants, osmotic agents, acidic or alkaline excipients for adjusting and/or buffering the pH, viscosity-increasing agents etc.

Stabilisers may be useful if the active ingredient of the gel composition is a protein sensitive to degradation. Examples of potentially suitable stabilisers include saccharides such as sugars and sugar alcohols, for example sucrose, trehalose, glucose, fructose, mannose, mannitol, sorbitol, and xylitol; amino acids such as glycin, histidine, aspartic acid, alanine, glutamic acid. Other common stabilisers are glycerol, albumin, gelatin, and polysorbate.

Examples of potentially suitable antioxidants include vitamin E or vitamin E derivatives, ascorbic acid, ascorbic acid esters, sulphites such as sodium bisulphite or acetone sodium bisulfite, hydrogen sulphites, gallic acid esters, butyl hydroxyanisole, butyl hydroxytoluene, and methionine.

Common preservatives which may potentially be suitable even for parenteral products include benzyl alcohol, chlorobutanol, methylparaben, propylparaben, phenol, m-cresol, and combinations of any of these. Of course, if the intended use of the gel composition does not involve parenteral, pulmonary or ophthalmic administration, a number of further preservatives known to a person skilled in the art may also be used.

Optional surfactants include poloxamers, polysorbates, phospholipids, vitamin E-TPGS, and macrogol hydroxystearates such as macrogol-15-hydroxystearate. Osmotic agents may be selected, for example, from salts, such as non-toxic inorganic salts; sugars and sugar alcohols, such as sucrose, trehalose, glucose, fructose, mannose, mannitol, sorbitol, and xylitol; amino acids such as glycin, histidine, aspartic acid, alanine, glutamic acid; or any other well-tolerated soluble excipient. A particularly preferred osmotic agent is sodium chloride. Acidic or alkaline excipients for adjusting and/or buffering the pH may be optionally selected from any commonly used organic and inorganic salts, acids, and bases. Viscosity-increasing agent are e.g. the gel-forming agents described further above.

The volume of liquid should be selected in consideration of the design and properties of the microparticles, such as to ensure that the aqueous gel having the desired gel strength is formed upon combination with the microparticles. Another factor to take into account is the intended use of the composition. For example, if the gel is to be injected subcutaneously or intramuscularly, the volume of the liquid should not be larger than about 5 ml, unless the kit is designed for the preparation of a multi-dose composition. More preferably, the volume is not more than about 3 ml, or not more than about 2 ml, respectively. In a further embodiment, the volume of the liquid is from about 0.3 to about 2 ml, such as about 0.5 ml or about 1 ml.

The liquid may optionally comprise the active agent which is to be incorporated into, and delivered by the gel composition. In particular, if it is not needed or desired that the active compound is encapsulated within the microparticles, this is a viable option. One of the advantages of this embodiment is that the active compound does not have to undergo the manufacturing conditions needed for making the microparticles. For example, it is possible that a particular drug candidate is potentially adversely affected by the chemical or physical agents used to effect or initiate the crosslinking of the hydrophilic polymer in the preparation of the particles.

If the active compound is not designed to be incorporated within the microparticles, and if it is not stable enough to be incorporated within the aqueous liquid carrier and retain its activity over a commercially desirable shelf life, it is another option that the method of making the gel composition further comprises a step of providing a solid component which comprises the active compound and, optionally, one or more excipients. For example, this solid component may be a lyophilised unit which may optionally comprise one or more bulking agents, lyophilisation aids, stabilisers, surfactant, suspension agents and the like. Alternatively, the active compound may be provided as a free flowing powder component, optionally comprising one or more excipients as appropriate.

According to this group of embodiments, at least three components are combined with each other to obtain the gel composition: the microparticle-containing component, the aqueous liquid, and the component comprising the active-compound. The components may be combined simultaneously or sequentially.

In another aspect, the invention provides a kit for the preparation of a viscoelastic aqueous gel composition. The kit comprises the component which are to be provided in analogy to the method described above. Such a kit may comprise a dry solid component and an aqueous liquid for reconstituting the gel composition, wherein the solid component preferably comprises the microparticles capable of gel formation or, in the case of gel compositions formed by two species of microparticles, at least one of the respective microparticle species.

More specifically, the kit preferably comprises a solid composition comprising microparticles based on a crosslinked hydrophilic polymer. The microparticles further have a core region and a surface region, and at least the surface region comprises hydrophobic domains formed by oligomeric side chains which are predominantly composed of monomeric units selected from L-lactide, D-lactide, glycolide, and combinations thereof.

With respect to the further preferred design and properties of the microparticles, reference is made to their disclosure in the context of the description of the gel composition herein-above. The solid composition may, for example, represent a lyophilisate or a flowable powder. It may further comprise any of the solid excipients disclosed above, such as one or more bulking agents, lyophilisation aids, stabilisers, surfactant, suspension agents, antioxidants, preservatives, surfactants, osmotic agents, acidic or alkaline excipients for adjusting and/or buffering the pH, viscosity-increasing agents etc.

The solid component may in fact comprise all solid excipients required for the aqueous gel composition. In this case, it may be reconstituted or converted into the gel simply by the addition of an appropriate volume of water, such as sterile water for injection. The measured amount of water may or may not be provided within the kit.

Alternatively, the solid component does not comprise any or all of the further solid excipients of the gel composition. In this case, it is preferred that the kit comprises a further component, which is an aqueous liquid which is adapted for reconstituting the gel. The aqueous liquid preferably comprises the further required excipients in dissolved form.

An active compound may be comprised in the solid composition of the kit which also comprises the microparticles. As described herein, the active compound may be incorporated within the microparticles or within a fraction or species of the microparticles if the particles consist of more than one fraction or species. Alternatively, the active compound is not embedded or encapsulated within the microparticles, but nevertheless represents a constituent of the solid composition.

Alternatively, or additionally, an active compound is incorporated within the liquid, or as a separate solid component of the kit.

The kit may represent a drug product, medicinal product, or a diagnostic product.

Further embodiments will become obvious from the following examples which illustrate the invention in some of its major aspects, without limiting the scope thereof.

### Example 1: Preparation of microparticles with hydrophobic domains

Dextran hydroxyethyl methacrylate (dex-HEMA) was prepared as described in WO 98/00170. The degree of substitution DS, i.e. the number of HEMA groups per 100 glucopyranose units, was approx. 10.

Subsequently, substantially spherical microparticles were obtained through radical polymerisation of the dex-HEMA, emulsified in an aqueous PEG solution, as described by Franssen et al. (Int J Pharm 1998;168:1-7). The mean volume diameter of the microparticles was approximately 10 µm. In the hydrated state, the particles had a water content of about 70 wt.-%.

L- and D-lactic acid oligomers were synthesised by a ring-opening polymerization reaction of lactide with ethyl lactate as initiator, according to De Jong et al. (Macromolecules 1998;31(19):6397-6402). The average degree of polymerization (DPₐᵥ) of the thus formed oligolactate was controlled by the ethyl lactate/lactide ratio. The DPₐᵥ values of the particles selected in this study were approx. 5 and 13, respectively.

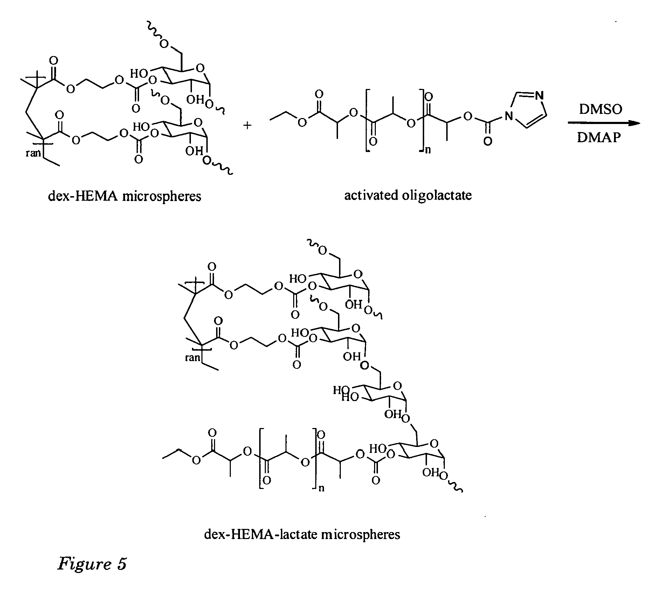

To couple the oligomers to dex-HEMA microspheres, the hydroxyl group of the oligomers was activated using *N,N'*-carbonyldiimidazole (CDI). The coupling reaction was in analogy to that described for lactate grafting of dextran (De Jong et al. Macromolecules 2000;33(10):3680-3686). In short, lyophilised dex-HEMA microspheres (500 mg) were dispersed in dry DMSO (10 mL). Next, DMAP (100 mg) was dissolved in the mixture. Subsequently, ethyl lactate-carbonyldiimidazole (e.g. 230 mg to obtain a theoretical DS of 8) was added and the vials were placed on a roller bench for 10 days. Finally the microspheres were washed 3 times with dichloromethane (DCM) and dried overnight by evaporation of the DCM. Figure 5 shows a schematic representation of the chemical reaction.

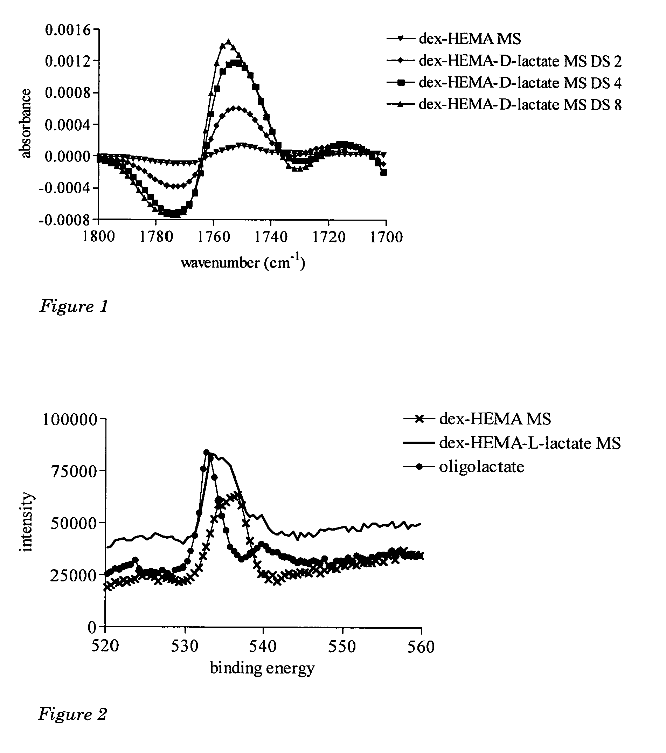

FTIR was used to confirm that lactate chains are present in/on the dex-HEMA microspheres. Microsphere dispersions were prepared (5 % w/w in Hepes buffer, 100 mM, pH 7) of which 10 µl was brought into a CaF₂ liquid cell. For this part of the experiment, microparticles with a DPₐᵥ of the oligolactate grafts of 13 were selected, while the oligolactate substitution degree was varied (DS_{lactate} 2, 4 and 8). Dex-HEMA microparticles show a carbonyl peak around 1750 cm⁻¹, and as lactate grafting increases the amount of carbonyl groups, the carbonyl peak should increase. Indeed, the grafting of dex-HEMA microparticles with oligolactate chains when conducted as described in this example results in a clear increase of this peak, confirming the actual presence of the oligolactate. These results are also depicted in figure 1.

To investigate the grafting efficiency of oligolactate-CI to the dex-HEMA microspheres, the lactic acid content of degraded microspheres was measured. Dex-HEMA-lactate microspheres (10 mg) were hydrolysed in alkaline conditions (1 ml of 0.1 N NaOH) and subsequently an equal amount of HCl was added (100 µl of 1 N HCl). Analysis of the amount of lactic acid in the samples was carried out with HPLC (Alltech Prevail Organic acid column (5 µm, 250 x 4.6 mm), using KH₂PO₃ buffer (25 mM, pH 2.5) and acetonitrile/water (95/5 w/w) as eluent A and B, respectively, at a flow rate of 1 ml/min. A gradient was run from 0 to 60 % B in 10 min, followed by an elution with 60 % B for 10 min, with a total runtime of 30 min. The injection volume was 50 µl and the detection wavelength was 210 nm. A calibration curve was linear between 0.01 and 4 µmol lactic acid.

The results revealed that only approximately 10-13 % and 16-20 % (for DPₐᵥ 13 and 5, respectively) of the amount of the added activated oligolactate was grafted to the microspheres. Another 60 % of unreacted oligolactate was detected in the washing fractions.

To provide further evidence for the presence of oligolactate side chains at the surface region of the microparticles, X-ray photoelectron spectroscopy (XPS) was performed on a sample of microparticles with a lactide DPₐᵥ of 13 and a DS of 8, using dex-HEMA microparticles and oligolactate as controls. In result, the oligolactate sXPS pectrum contains a C=O peak (~ 532 eV), while the dex-HEMA microspheres show a peak originating from C-O (~ 536 eV). The spectrum of dex-HEMA-L-lactate microspheres overlaps the others, indicating the presence of both C-O and C=O groups, which means that the hydrophobic domains are indeed present on the surface of the particles.The respective data are depicted in figure 2.

### Example 2: Preparation of microparticles with non-covalently attached hydrophobic domains

Dex-HEMA microspheres were modified with non-CI-activated oligolactate (DPₐᵥ 13, amount added to obtain DS 8) through a procedure which was otherwise similar to the grafting method described in example 3 below. The microparticles thus treated comprised about 2.5 wt.-% of the oligolactide, which was determined by quantifying the lactic acid content after degrading the microspheres. This indicated that some non-covalent association occured, resulting in the presence of hydrophobic domains at the surface of the microspheres.

### Example 3: Preparation of viscoelastic gels from grafted microparticles

Microparticles from dextran hydroxyethyl methacrylate (dex-HEMA) grafted with oligo-L- or oligo-D-lactide grafts were prepared according to example 1. The approximate DPₐᵥ of the oligolactate chains was 5 and 13. The DS_{lactate} was varied between 2 and 8.

The dry microparticles were seperately dispersed in aqueous buffer solution (Hepes, 100 mM, pH 7) to form suspensions having a solid content of 12.5 and 15 wt.-%, respectively. The suspensions were allowed to hydrate overnight at 4 °C. Gel formation was observed for all samples.

The viscous gels were characterised by rheological measurements using a controlled stress rheometer (AR1000-N, TA Instruments, Etten-Leur, The Netherlands) equipped with an acrylic flat plate geometry (20 mm diameter) and a gap of 500 µm. A solvent trap was used to prevent evaporation of the solvent. The viscoelastic properties of the samples were determined by measuring the G' (shear storage modulus) and G" (loss modulus) at 20 °C with a constant strain of 1 % and constant frequency of 1 Hz. Creep experiments were performed to evaluate the extent of recovery of the material after deformation. In the creep experiment a shear stress of 10 Pa was applied while the strain was monitored. After 1 min the stress was removed and the recovery of the sample was monitored by measuring the strain during 2 min.

Apparently, both types of microspheres (i.e. grafted with either oligo-L- or oligo-D- lactate) were able to interact with each other in aqueous suspension, forming a threedimensional network resulting in a viscoelastic gel. The increase of the solid content lead to stronger gels, as well as increasing the grafting density (DS) and DPₐᵥ of the oligolactate chains. Table 1 shows the storage modulus values (G' (Pa)) found for the samples with a solid content of 15 wt.-%. Creep experiments showed that the networks were predominantly elastic.

**Table 1**

| Sample | DPₐᵥ | DS | Side chains | G' (Pa) |
|---|---|---|---|---|
| A | 5 | 8 | oligo-L-lactate | 740 |
| B | 5 | 8 | oligo-D-lactate | 890 |
| C | 13 | 2 | oligo-L-lactate | 790 |
| D | 13 | 2 | oligo-D-lactate | 580 |
| E | 13 | 8 | oligo-L-lactate | 1920 |
| F | 13 | 8 | oligo-D-lactate | 2400 |

### Example 4: Preparation of viscoelastic gels from grafted microparticles capable of forming stereocomplexes

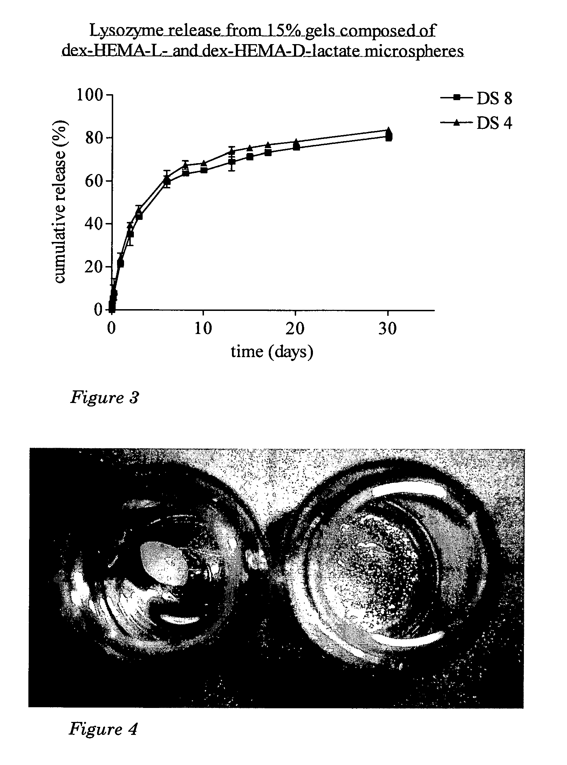

In this experiment, equal amounts of dry dex-HEMA microparticles with oligo-L-lactide side chains and dex-HEMA microparticles with oligo D-lactide side chains, both prepared according to example 1, were combined and dispersed in buffer as described in example 3, resulting in the formation of viscoelastic gels. A typical gel obtained from this procedure is shown in figure 4 (left).

Rheological analysis was performed as described in example 3. In comparison to the sample of example 3 having similar solid content, DP, and DS, these gel networks were generally stronger, indicating the presence of an additional type of interaction between the microparticles which is believed to be stereocomplex formation.

As for solely dex-HEMA-L-lactate or dex-HEMA-D-lactate microspheres, a higher DS_{lactate} lead to a stronger network. For example, gels with a solid content of 15 wt.-% and a DPₐᵥ of 13 exhibited G'values of about 1400 Pa, 2900 Pa, and 4400 Pa when the DS was 2, 4, and 8, respectively. At a lower DPₐᵥ of 5 instead of 13, the gel network was less strong. For example, at a solid content of 15 wt.-% and a DS of 8, G' was approx. 1100 Pa.

Moreover, in view of the application of the system as injectable composition, the possibility to flow was investigated in stress sweep experiments at 20 °C. During these experiments the G' and G" were monitored while the stress was increased. The frequency was kept constant at 1 Hz. The yield point was defined as the point at which the system started to flow, more precisely, the srress at which the tan (δ) rose above 1.

In result, yield points ranging from about 10 Pa for gels comprising the stereocomplex-forming microparticle mixture with a solid content of 12.5 wt.-%, a DS of 2 and a DPₐᵥ of 13 to about 1,200 Pa for gels with the same solid content and DPₐᵥ, but with a DS of 8, were found.

### Example 5: Preparation of viscoelastic gels from microparticles with non-covalently attached hydrophobic domains

In this experiment, equal amounts of dry dex-HEMA microparticles with non-covalently attached oligo-L-lactide and dex-HEMA microparticles with non-covalently attached oligo-D-lactide, both prepared according to example 2, were combined and dispersed in buffer as described in example 3. In another series of tests, the microparticles were hydrated separately. In all cases, viscoelastic gels were formed, with G' values of approximately 600 Pa for the separately hydrated microparticles, whereas the microparticle mixture capable of forming stereocomplexes formed a stronger network with a storage modulus of approx. 900 Pa.

### Example 6: Release of an active agent

Viscoelastic gel compositions with a solid content of 15 wt.-% comprising lysozyme as a model active compound were prepared by dispersing equal amounts of dex-HEMA-L-lactate and dex-HEMA-D-lactate microspheres (DS 4 and 8) in lysozyme-containing (1 wt.-%) aqueus buffer solution. The microparticles were allowed to hydrate overnight at 4 °C. Viscoelastic gels were formed which were then tested for lysozyme release using the method described by Van Tomme et al. JCR 2005;110:67-78. In short, samples of 500 mg of the gels were transferred in a release device and 3 mL release buffer (Hepes, pH 7, 100 mM, 150 mM NaCl, 0.02 % NaN₃). At certain time intervals, samples were taken and analysed for their protein content using a BCA® protein assay. After approximately one month, about 80 % of the entrapped lysozyme was released. No significant difference could be detected between the two batches (i.e. with DS 4 and DS 8) were observed. The release profiles are shown in figure 3.

### Example 7 (comparative example): Aqueous suspension of poly-L-lactide or poly-D-lactide microparticles

Microparticles essentially consisting of either poly-L-lactide or poly-D-lactide were prepared by means of an oil-in-water solvent evaporation process. The respective polymer was dissolved in chloroform. An emulsifier (PVA) was dissolved in water to provide the aqueous phase. While the water phase was vigorously stirred, the polymer-containing oil phase was added dropwise, creating an oil-in-water emulsion. Subsequent evaporation of the organic solvent was performed to harden the polymer droplets. leading to the formation of microspheres. The microparticles were harvested by multiple washing and centrifugation steps. Subsequently, the microparticles were freeze-dried.

Equal amounts of the two types of microparticles were dispersed in aqueous buffer to form a suspension with a solid content of 15 wt.-%. In contrast to example 4, no gel formation was observed (figure 4, right).

## Claims

1. A viscoelastic aqueous gel composition comprising microparticles having a core region and a surface region, wherein the microparticles comprise a crosslinked hydrophilic polymer, and wherein at least the surface region of the microparticles comprises hydrophobic domains.

2. The composition of claim 1, wherein the viscoelasticity of the aqueous gel is effected by the content of the microparticles.

3. The composition of claim 1 or 2, wherein the hydrophobic domains are covalently attached to the crosslinked hydrophilic polymer.

4. The composition of any one of claims 1-3, wherein the hydrophobic domains are oligomeric or polymeric side chains to the crosslinked hydrophilic polymer.

5. The composition of, claim 4 wherein the oligomeric or polymeric side chains are predominantly composed of monomeric units selected from L-lactide, D-lactide, glycolide, caprolactone, trimethylene carbonate, and combinations thereof.

6. The composition of any one of claim 1-5, comprising a first and a second species of microparticles, wherein the microparticles of both species have a core region and a surface region, and wherein the microparticles of both species comprise a crosslinked hydrophilic polymer, and wherein at least the surface region of the microparticles of both species comprise oligomeric or polymeric side chains which are predominantly composed of monomeric units selected from L-lactide, D-lactide, glycolide, and combinations thereof, further **characterised in that** oligomeric side chains of the first species of microparticles are capable of forming stereocomplexes with oligomeric side chains of the second species of microparticles.

7. The composition of any of claims 1-6, wherein the hydrophobic domains comprise in average from 2 to about 20 monomeric units per side chain.

8. The composition of any of claims 1-6, wherein the degree of substitution relative to the hydrophilic polymer is in the range from about 0.5 to about 20.

9. The composition of any of the preceding claims, wherein the hydrophilic polymer is selected from optionally derivatised polysaccharides including dextran, starch, cellulose, alginate, pectin, chitosan, hyaluronic acid; optionally derivatised polypeptides including albumin, lysozyme, poly(amino acids), including poly(lysine) collagen, including copolymers thereof, poly(glutamic acid) and copolymers thereof; poly(acrylates) and/or poly(acrylamides), poly(alkyl acrylates)/(alkyl acrylamides) such as poly(methacrylate), poly(hydroxyethyl methacrylate), poly(hydroxypropyl methacrylate), poly(hydroxyethyl methacrylamide), poly(hydroxypropyl methacrylamide); poly(vinylalcohol), poly(ethylene glycol), water soluble polyphosphazenes, and mixtures thereof.

10. The composition of claim 9, wherein the hydrophilic polymer is selected from dextran derivatives, preferably dextran hydroxyethyl methacrylate, dextran hydroxypropyl methacrylate, dextran hydroxyethyl methacrylamide, and dextran hydroxypropyl methacrylamide.

11. The composition of any of the preceding claims, wherein the hydrophilic polymer comprises at least one bond which is hydrolysable under physiological conditions, which bond is preferably a peptide, carbonate, lactate, glycolate, or succinate ester bond.

12. The composition of any of the preceding claims, being further **characterised in that** it is substantially free of dissolved or colloidally dispersed gel-forming agents.

13. The composition of any of the preceding claims, comprising a bioactive compound which is preferably selected from the group consisting of peptides, proteins, antigens, vaccine adjuvants, antibodies, antibody fragments, nucleotides, iRNA, siRNA, hormones, cytostatic or cytotoxic agents, agents acting on the central nervous system, living cells, fragments of living cells, and pluralities of living cells.

14. The composition of claim 13, wherein at least a portion of the bioactive compound is entrapped within the microparticles.

15. The composition of claim 13, wherein at least a portion of the bioactive compound is entrapped between the microparticles.

16. The composition of any of the claims 13 to 15, wherein the bioactive compound is released over a period ranging from about 1 day to about 6 months under physiological conditions.

17. The composition of any of the preceding claims, wherein the microparticles have a weight average diameter from about 1 to about 50 µm.

18. The composition of any of the preceding claims, having a microparticle content from about 5 to about 50 wt.-%.

19. The composition of any of the preceding claims, having viscoelastic properties with a yield point of at least about 50 Pa.

20. The composition of any of the preceding claims, being adapted for parenteral injection.

21. A microparticle having a core region and a surface region, said microparticle comprising a crosslinked hydrophilic polymer, wherein at least the surface region of the microparticle comprises hydrophobic domains formed by oligomeric side chains which are predominantly composed of monomeric units selected from L-lactide, D-lactide, glycolide, and combinations thereof.

22. The microparticle of claim 21, wherein at least some of the oligomeric side chains are composed of enantiomerically enriched monomeric units.

23. The microparticle of claim 21 or 22, wherein the hydrophobic domains comprise in average from 2 to about 20 monomeric units per side chain.

24. The microparticle of any of the claims 21 to 23, wherein the degree of substitution relative to the hydrophilic polymer is in the range from about 0.5 to about 20.

25. The microparticle of any of the claims 21 to 24, wherein the hydrophilic polymer is selected from optionally derivatised polysaccharides including dextran, starch, cellulose, alginate, pectin, chitosan, hyaluronic acid; optionally derivatised polypeptides including albumin, gelatin, collagen, lysozyme, poly(amino acids), including poly(lysine) including copolymers thereof, poly(glutamic acid) and copolymers thereof; poly(acrylates) and/or poly(acrylamides), poly(alkyl acrylates)/(alkyl acrylamides) such as poly(methacrylate), poly(hydroxyethyl methacrylate), poly(hydroxypropyl methacrylate), poly(hydroxyethyl methacrylamide), poly(hydroxypropyl methacrylamide); poly(vinylalcohol), poly(ethylene glycol), water soluble polyphosphazenes, and mixtures thereof.

26. The microparticle of claim 25, wherein the hydrophilic polymer is selected from dextran derivatives, preferably dextran hydroxyethyl methacrylate, dextran hydroxypropyl methacrylate, dextran hydroxyethyl methacrylamide, and dextran hydroxypropyl methacrylamide.

27. The microparticle of any of the claims 21 to 26, wherein the hydrophilic polymer comprises at least one bond which is hydrolysable under physiological conditions, which bond is preferably a carbonate, lactate, glycolate, or succinate bond.

28. The microparticle of any of the claims 21 to 27, comprising a bioactive compound which is preferably selected from the group consisting of peptides, proteins, antigens, vaccine adjuvants, antibodies, antibody fragments, nucleotides, iRNA, siRNA, hormones, cytostatic or cytotoxic agents, agents acting on the central nervous system, living cells, fragments of living cells, and pluralities of living cells.

29. The microparticle of any of the claims 21 to 28, having a weight average diameter from about 1 to about 50 µm.

30. A plurality of microparticles being substantially free of ionic charges, said microparticles being adapted to be capable of forming a viscoelastic gel in an aqueous environment.

31. A kit for the preparation of a viscoelastic aqueous gel composition, said kit comprising a solid composition comprising microparticles having a core region and a surface region, said microparticles comprising a crosslinked hydrophilic polymer, wherein at least the surface region of the microparticles comprises hydrophobic domains formed by oligomeric side chains which are predominantly composed of monomeric units selected from L-lactide, D-lactide, glycolide, and combinations thereof.

32. The kit of claim 31, wherein the solid composition comprises a first and a second species of microparticles, wherein the microparticles of both species have a core region and a surface region, and wherein the microparticles of both species comprise a crosslinked hydrophilic polymer, and wherein at least the surface region of the microparticles of both species comprise oligomeric side chains which are predominantly composed of monomeric units selected from L-lactide, D-lactide, glycolide, and combinations thereof, further **characterised in that** oligomeric side chains of the first species of microparticles are capable of forming stereocomplexes with oligomeric side chains of the second species of microparticles.

33. The kit of claim 31 or 32, further comprising an aqueous liquid which is adapted for reconstituting the gel composition.

34. A method for making a viscoelastic aqueous gel composition, said method comprising the steps of:
(a) providing microparticles having a core region and a surface region, wherein the microparticles comprise a crosslinked hydrophilic polymer, and wherein at least the surface region of the microparticles comprises hydrophobic domains;
(b) providing an aqueous liquid; and
(c) combining said microparticles and said aqueous liquid to obtain the viscoelastic gel.

35. The use of the composition of any of the claims 1 to 20 in the manufacture of a medicament, diagnostic product, or tissue engineering product.

36. The use of claim 35, wherein the medicament is for parenteral administration.

37. The use of claim 35 or 36, wherein the medicament is a depot formulation.

38. The use of any of the claims 35 to 37, wherein the medicament is a vaccine.
